# EUROPEAN PATENT APPLICATION

(11) **EP 4 672 263 A1**
(43) Date of publication of application: **31.12.2025**
(21) Application number: 24184766.4
(22) Date of filing: 26.06.2024
(51) Int. Cl.: G16H 50/30, G16H 50/70, G07C 5/08, B60R 21/01

(54) **METHOD AND SYSTEM FOR PREDICTING AN INJURY OF A VEHICLE PASSENGER, DATA PROCESSING APPARATUS, VEHICLE, COMPUTER PROGRAM, AND USE**

(71) Applicant: Volvo Car Corporation, 405 31 Göteborg (SE)
(72) Inventor: KOPPISETTY, Ashok Chaitanya, 40531 Göteborg (SE); LJUNG AUST, Mikael, 40531 Göteborg (SE)
(74) Representative: Maiwald GmbH

(57) **Abstract**

The disclosure relates to a method for predicting an injury of a vehicle passenger (14) caused by a crash event. The method comprises obtaining first data (D1) indicative of the crash event. Furthermore, the method comprises obtaining second data (D2) indicative of a plurality of historic crash events and at least one associated injury indicator (IH) for each of the historic crash events. Moreover, the method comprises selecting one item of the second data (D2) indicative of a selected historic crash event based on the first data (D1), and providing the at least one injury indicator (IH) associated with the selected historic crash event as a predictor (P) of the injury of the vehicle passenger (14) caused by the crash event. The present disclosure also relates to a data processing apparatus (20) and a system (12) for predicting an injury of a vehicle passenger (14). Additionally, the present disclosure is directed to a vehicle (10), and a computer program (28). Furthermore, the present disclosure relates do a use of second data (D2) indicative of a plurality of historic crash events and at least one associated injury indicator (IH) for each of the historic crash events.

## Description

### TECHNICAL FIELD

The present disclosure relates to a method for predicting an injury of a vehicle passenger caused by a crash event.

The present disclosure also relates to a data processing apparatus.

Additionally, the present disclosure is directed to a system for predicting an injury of a vehicle passenger caused by a crash event.

Moreover, the present disclosure relates to a vehicle.

The present disclosure is also directed to a computer program.

Furthermore, the present disclosure relates to a use of second data indicative of a plurality of historic crash events.

### BACKGROUND ART

Methods for predicting an injury of a vehicle passenger caused by a crash event are helpful for providing appropriate assistance, e.g. medical assistance, in a timely manner. The same applies to systems for predicting an injury of a vehicle passenger caused by a crash event. On the one hand, such methods and systems need to be able to provide the prediction of the injury relatively quickly. On the other hand, the prediction shall be precise.

### SUMMARY

Thus, it is an objective of the present disclosure to improve known methods and systems for predicting an injury of a vehicle passenger caused by a crash event such that the prediction is both available relatively quickly and precise. In other words, the above-mentioned conflict of objectives shall be alleviated or solved.

The problem is at least partially solved or alleviated by the subject matter of the independent claims of the present disclosure, wherein further examples are incorporated in the dependent claims.

According to a first aspect, there is provided a method for predicting an injury of a vehicle passenger caused by a crash event. The method comprises:
- obtaining first data indicative of the crash event,
- obtaining second data indicative of a plurality of historic crash events and indicative of at least one associated injury indicator for each of the historic crash events,
- selecting one item of the second data indicative of a selected historic crash event based on the first data, and
- providing the at least one injury indicator associated with the selected historic crash event as a predictor of the injury of the vehicle passenger caused by the crash event.

Thus, the present method uses data of historic crash events, wherein in this context, historic means dating from a past time. This data, more precisely the second data, characterizes the crash events as such and additionally comprises at least one associated injury indicator. In simplified words, the second data describe a plurality of crash events and the injuries the respective crash event has caused for a vehicle passenger. The second data may be provided in a database or any other form suitable for selecting one element or item of the second data. As mentioned before, the one item of the second data is selected based on the first data. The first data are indicative of an actual, current and/or imminent crash event. Thus, the first data may be real time data collected by the vehicle undergoing the crash event and/or from the perspective of the vehicle undergoing the crash event. This can also be described in that the selection of the one item of the second data is a function of the first data or at least depends on the first data. Since the at least one injury indicator of the selected historic crash event as described by the selected one item of the second data is used as a predictor of the injury of the vehicle passenger caused by the actual, current and/or imminent crash event, the selection of the one item of the second data is performed such that the selected historic crash event of the second data is similar or at least a suitable approximation of the crash event as described by the first data. Consequently, the injury indicator of the selected historic crash event as described by the selected item of the second data, is a good, i.e. sufficiently precise, predictor of the injury of the vehicle passenger caused by the crash event. At the same time, selecting one item of the second data is computationally simple and, therefore, may be executed within a comparatively short period of time, i.e. quickly.

It is understood that in the context of the present disclosure, a vehicle passenger can be a driver, a front seat passenger and/or a rear-seat passenger.

Moreover, in the context of the present disclosure, obtaining means receiving or determining.

In the context of the present disclosure, the predictor of the injury of the vehicle passenger may as well be called a prediction of the injury of the vehicle passenger.

The predictor or prediction of the injury of the vehicle passenger caused by the actual, current and/or imminent crash event may be transmitted to a service entity, e.g. a medical service provider or a traffic regulation authority. Consequently, based on the predictor or prediction, timely and appropriate medical assistance may be provided to the vehicle passenger. Moreover, timely and appropriate actions in traffic management may be taken, e.g. traffic lights may be operated in order to allow the medical service provider to reach the injured vehicle passenger without further delay, intersections may be blocked, etc.

The selection of the item of the second data may be done using a look-up process and/or a mapping technique.

According to an example, the second data is indicative of at least twenty different historic crash events and at least one associated injury indicator for each of the historic crash events. In another example, the second data is indicative of at least fifty different historic crash events and at least one associated injury indicator for each of the historic crash events. In still another example, the second data is indicative of at least a hundred different historic crash events and at least one associated injury indicator for each of the historic crash events. In another example, the second data is indicative of at least 150 different historic crash events and at least one associated injury indicator for each of the historic crash events. In all of these examples, a sufficiently precise predictor of the injury of the vehicle passenger caused by the crash event may be provided. It is understood that the more historic crash events and associated injury indicators are described by the second data, the more precise the predictor may be. The above-mentioned historic crash events may be physical crash events. Optionally, these physical crash events may be complemented by virtual historic crash events. In this context, one thousand or more virtual historic crash events may be used. In an example, several thousand virtual historic crash events are used.

According to an example, the first data is indicative of one or more of an impact force, an angle of collision, a vehicle deformation and a speed at the moment of impact. The speed at the moment of impact is a speed of the vehicle involved in the crash event. Using one or more of the impact force, the angle of collision, the vehicle deformation and a speed at the moment of impact allows to precisely describe or characterize a crash event. Based thereon, an appropriate item of the second data may be selected with high confidentiality. Consequently, the precision of the predictor or prediction of the injury is further enhanced.

Additionally or alternatively, the second data is indicative of one or more of an impact force, an angle of collision, a vehicle deformation and a speed at the moment of impact for each of the plurality of historic crash events. The speed at the moment of impact is a speed of the vehicle involved in the crash event. As has been mentioned before, using one or more of the impact force, the angle of collision, the vehicle deformation and a speed at the moment of impact allows to precisely describe or characterize a crash event, in the present case a historic crash event. Based thereon, an appropriate selection may be facilitated. Consequently, the precision of the predictor or prediction of the injury is further enhanced.

In the context of the present disclosure, the impact force may be measured directly by a suitable system of the vehicle undergoing the crash event. Additionally or alternatively, the impact force may be measured indirectly. In an example, the impact force may be derived from a measured deceleration of the vehicle undergoing the crash event. In another example, the impact force may be calculated based on a number of acceleration data points obtained from a number of associated acceleration sensors located at different positions on the vehicle. In this context, a so-called crash pulse may be determined which corresponds to a peak acceleration during impact. Optionally, also impact data points obtained from impact sensors may be used. In another example, the impact force may be derived using a simulation model. Additionally or alternatively, a collision object, i.e. an object colliding with the vehicle, may be detected and classified. This may further enhance the precision of the determination of the impact force.

Also the angle of collision may be measured directly by a suitable system of the vehicle undergoing the crash event. Additionally or alternatively, the angle of collision may be measured indirectly. Similar to the above, the angle of collision may be determined based on a number of acceleration data points obtained from a number of associated acceleration sensors located at different positions on the vehicle. Optionally, also impact data points obtained from impact sensors may be used. Since the acceleration sensors and/or the impact sensors have a known position and sensitivity direction, one way to determine the angle of collision is to calculate a vector average of the impact forces on these sensors using trigonometry. Additionally or alternatively, a collision object, i.e. an object colliding with the vehicle, may be detected and classified. This may further enhance the precision of the determination of the angle of collision.

The vehicle deformation may as well be measured directly by a suitable system of the vehicle undergoing the crash event. Additionally or alternatively, it is possible to measure the vehicle deformation indirectly. In an example, the vehicle deformation is determined based on a known impact force and a known impact force direction. Additionally or alternatively, the vehicle deformation may be determined based on a known impact force and information from previous crash testing. The information from previous crash testing comprises an information on an impact force and a resulting deformation. In the above examples, the known impact force may be associated with a known impact force direction. In another example, one or more sensors located on the vehicle may be used to determine vehicle deformation. Examples of such sensors include cameras and ultrasound sensors. It is understood that these sensors need to be operational in order to determine the vehicle deformation based thereon, i.e. the sensors must not be affected by the crash.

Also the speed of the vehicle at the moment of impact may be measured directly by a suitable system of the vehicle, e.g. a speedometer. This means that the speed of the vehicle is determined based on a known size of the wheels and a rotational speed of at least one wheel. Additionally or alternatively, the speed of the vehicle at the moment of impact may be measured indirectly, e.g. using a time series of GPS positions.

In an example, the injury indicator is indicative of one or more of a type of injury, an injury severity and one or more regions of the human body. In this context, a type of injury describes a type of physiological damage that is caused in the body of the vehicle passenger. An injury severity describes an extent, a level or intensity of the physiological damage that is caused in the body of the vehicle passenger. The regions of the human body may be defined in several ways. In one example, the body is subdivided into the legs, the torso, the head and the arms. Of course, it is also possible to further subdivide these body parts. In another example, the so-called classification according to the Abbreviated Injury Scale (AIS) may be used. This is an anatomical-based coding system created by the Association for the Advancement of Automotive Medicine to classify and describe the severity of injuries. AIS captures the threat to life associated with a particular injury, i.e. a type of injury. It is noted that the type of injury, the injury severity and the regions of the human body may be used alone or in combination in order to describe the injury, i.e. in order to form the injury indicator. Thereby, the injury of the vehicle passenger caused by the historic crash event may be described in a precise manner. Based thereon, an appropriate predictor may be derived and appropriate medical assistance may be provided. This is for example due to the fact that based on the injury indicator the medical assistance may bring the suitable type of equipment and/or can precisely estimate the urgency.

In an example, the second data is crash test data. In other words, the second data is indicative of historic crash events which have happened in a test environment. Such historic crash events may have happened during the development of the vehicle. Moreover, it is usual and even necessary to conduct a plurality of crash tests during the development of a vehicle. The crash tests may be physical and/or virtual. The data produced during these crash events may be used as second data. Also in this example, the second data may be provided in the form of a database. Thus, in this example, the database comprises a plurality of crash test results which may have been obtained during the development of the vehicle.

According to an example, the first data is associated with a predefined make and model of a vehicle. The second data is associated with the same predefined make and model of the vehicle. In this context, a vehicle's make it is the company that made it. The vehicle's model is the vehicle's specific name used for distinguishing the vehicles of the same make. If the first data and the second data are associated with a vehicle of the same make and model, the first data and the second data are highly comparable. This means that the at least one injury indicator associated with the selected historic crash event is a highly precise predictor of the injury of the vehicle passenger caused by the crash event.

In an example, the method further comprises pre-processing the first data before selecting one item of the second data indicative of a selected historic crash event based on the first data. In this context, pre-processing the first data means that the first data are manipulated, filtered and/or augmented before the item of the second data is selected based on the first data. Due to the pre-processing, the selection of the item of the second data may be facilitated since the pre-processed first data may allow for an easier, quicker and/or clearer selection of the item of the second data that is similar or at least a suitable approximation. In an example, pre-processing the first data reduces or eliminates noise comprised by the first data. More generally speaking, pre-processing the first data facilitates the comparison of the first data to the second data such that the most appropriate item of the second data may be reliably selected.

In a case in which the second data is crash test data, pre-processing the first data allows to compensate the differences between a crash test, i.e. a crash event that happens in a controlled test environment, and a real-life crash event. This further facilitates the selection of an appropriate item of the second data.

According to an example, the pre-processing comprises using a machine learning technique and/or an artificial intelligence model. Based thereon, pre-processing the first data may be done in a manner allowing for a good compromise between a short calculation time and a precise result of the pre-processing. Examples could include Siamese Networks and Triplet Loss.

According to an example, selecting one item of the second data indicative of a selected historic crash event comprises using a pattern matching technique. Thus, using the pattern matching technique, the item of the second data may be selected which is most similar to the first data. Based thereon, a precise predictor of the injury of the vehicle passenger may be provided.

In the context of using a pattern matching technique, sensor signals may optionally be broken down into discrete states of the vehicle. In such a case, the vehicle and, thus, the vehicle states, may be describes using a spring analogy, i.e. the vehicle and the vehicle states may be described as a plurality of interconnected springs and states of a plurality of interconnected springs.

An example of a pattern matching technique is a so-called continuous sensor time series similarity pattern matching. In this case, time series of sensor detection results, e.g. indicative of one or more of an impact force, an angle of collision, a vehicle deformation and a speed at the moment of impact, are compared with the objective to find a time series of sensor detection results in the second data that is most similar to the time series of sensor detection results in the first data. Additionally or alternatively, other pattern matching algorithms can be used. Examples of such methods that are suitable in this case are dynamic time warping (DTW), Euclidean distance, Hamming distance, Levenstein distance, longest subsequence and cosine similarity. The previously mentioned methods are primarily based on vector operations that are architecturally feasible and performed on binary data.

In an example, the method further comprises:
- obtaining a first similarity metric based on the first data,
- obtaining a plurality of second similarity metrics based on the second data, each second similarity metric being associated with one of the plurality of historic crash events, and
- selecting one item of the second data indicative of a selected historic crash event based on the first similarity metric and the second similarity metrics.

In an example, the similarity metric may be expressed as a percentage. In another example, cosine similarity is used as a similarity metric. Consequently, the item of the second data having the highest similarity to the first data may be selected and the associated injury indicator may be used as the predictor of the injury of the vehicle passenger caused by the crash event.

According to an example, the method further comprises
- obtaining third data indicative of a physical condition of the vehicle passenger, and
- selecting one item of the second data indicative of a selected historic crash event based on the third data.

Third data may be provided by a portable electronic device of the vehicle passenger, e.g. a smart phone or smart watch or another wearable device. Additionally or alternatively, the third data may be provided by a passenger monitoring system. Such a system for example comprises a camera or radar unit configured to provide third data. Consequently, the item of the second data is selected not only based on the first data, i.e. the data indicative of the crash event, but also based on the third data. This allows for an even more reliable selection of the most suitable item of the second data, such that a highly precise predictor of the injury of the vehicle passenger may be provided. In a case in which the third data is provided by a camera, a physical condition and/or a movement of the vehicle passenger may be taken into account when selecting the item of the second data.

In another example, the third data is indicative of which seats are occupied inside the vehicle. Moreover, the third data may be indicative of an estimate of one or more of an occupant posture, occupant weight and occupant height. In this case, the third data may be provided by an occupancy sensor and/or weight sensor installed in the relevant seat. Alternatively, a passenger monitoring system, e.g. comprising a camera, may be used. This allows for an even more reliable selection of the most suitable item of the second data, such that a highly precise predictor of the injury of the vehicle passenger may be provided.

In an example, the item of the second data which is selected based on the first data is validated or verified based on the third data. In this example, a plausibility check may be performed using the third data.

According to an example, the method further comprises updating the second data based on the first data and based on at least one associated injury indicator. Thus, the first data and the associated injury indicator are transformed into an additional item of the second data. If the second data is provided in the form of a database, the first data and the associated injury indicator form an additional entry in this database. As has been mentioned before, the more entries such a database comprises, the better the predictor for an injury of the vehicle passenger. Consequently, updating the second data enhances the quality of the predictor.

The method may be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise one or more of a processor, a memory, a data interface, or the like.

According to an example, the method of the present disclosure is fully or partially executed by so-called in-memory calculation or in-memory computation. This means that some or all steps of the method of the present disclosure are executed by in-memory computation. In-memory computation is a computational paradigm that addresses the von Neumann bottleneck in the traditional computational architectures. In the von Neumann architecture, the central processing unit contains the control unit, arithmetic logic unit and registers. The processor and memory are separate components, with data moving between them via the system bus that has a bottleneck with increasing data loads and or demanding computations. This could be solved by performing computations directly within the memory arrays and thus minimizing or eliminating the need for frequent data transfer between the central processing unit and the memory. Thus, the method of the present disclosure may be executed in a computationally efficient manner.

In an example, a phase change memory may be used for doing the in-memory computation. Phase Change Memory (PCM) is a type of non-volatile memory that has favorable material properties for the execution of the method of the present disclosure. The materials constituting the memory can switch between the amorphous and crystallin states that have distinct electrical resistances enabling storage of binary data. Phase change memory devices are non-volatile, scalable and highly durable. Implementing continuous sensor time series similarity pattern matching directly on a PCM device using in-memory computation involves leveraging the PCM's ability to perform computations directly within the memory array, reducing the need to transfer data to and from a separate processing unit. This can significantly speed up the process and enhance the efficiency of pattern matching. In addition to the speed due to the inherent properties of PCM materials such an apparatus performing pattern matching is scalable and highly durable to external forces.

According to a second aspect, there is provided a data processing apparatus. The data processing apparatus comprises means for carrying out the method of the present disclosure. Thus, using such a data processing apparatus, data of historic crash events may be used, wherein in this context, historic means dating from a past time. This data, more precisely the second data, characterizes the crash events as such and additionally comprises at least one associated injury indicator. In simplified words, the second data describe a plurality of crash events and the injuries the respective crash event has caused for a vehicle passenger. The second data may be provided in a database or any other form suitable for selecting one element or item of the second data. As mentioned before, the one item of the second data is selected based on the first data. The first data are indicative of an actual, current and/or imminent crash event. Thus, the first data may be real time data collected by the vehicle undergoing the crash event and/or from the perspective of the vehicle undergoing the crash event. This can also be described in that the selection of the one item of the second data is a function of the first data or at least depends on the first data. Since the at least one injury indicator of the selected historic crash event as described by the selected one item of the second data is used as a predictor of the injury of the vehicle passenger caused by the actual, current and/or imminent crash event, the selection of the one item of the second data is performed such that the selected historic crash event of the second data is similar or at least a suitable approximation of the crash event as described by the first data. Consequently, the injury indicator of the selected historic crash event as described by the selected item of the second data, is a good, i.e. sufficiently precise, predictor of the injury of the vehicle passenger caused by the crash event. At the same time, selecting one item of the second data is computationally simple and, therefore, may be executed within a comparatively short period of time, i.e. quickly.

According to a third aspect, there is provided a system for predicting an injury of a vehicle passenger caused by a crash event, the system comprises
- a data processing apparatus of the present disclosure,
- at least one sensor configured for providing first data indicative of a crash event, wherein the sensor is communicatively connected to the data processing apparatus,
- a data storage means storing second data indicative of a plurality of historic crash events and at least one associated injury indicator for each of the historic crash events, wherein the data storage means is communicatively connected to the data processing apparatus or forms part of the data processing apparatus, and
- an output interface for providing the at least one injury indicator as a predictor of the injury of the vehicle passenger caused by the crash event, wherein the output interface is communicatively connected to the data processing apparatus.

Thus, using such a system, data of historic crash events may be used, wherein in this context, historic means dating from a past time. This data, more precisely the second data, characterizes the crash events as such and additionally comprises at least one associated injury indicator. In simplified words, the second data describe a plurality of crash events and the injuries the respective crash event has caused for a vehicle passenger. The second data may be provided in a database, i.e. the data storage means may be a database, or any other form suitable for selecting one element or item of the second data. As mentioned before, the one item of the second data is selected based on the first data. The first data are indicative of an actual, current and/or imminent crash event. Thus, the first data may be real time data collected by the vehicle undergoing the crash event and/or from the perspective of the vehicle undergoing the crash event. This can also be described in that the selection of the one item of the second data is a function of the first data or at least depends on the first data. Since the at least one injury indicator of the selected historic crash event as described by the selected one item of the second data is used as a predictor of the injury of the vehicle passenger caused by the actual, current and/or imminent crash event, the selection of the one item of the second data is performed such that the selected historic crash event of the second data is similar or at least a suitable approximation of the crash event as described by the first data. Consequently, the injury indicator of the selected historic crash event as described by the selected item of the second data, is a good, i.e. sufficiently precise, predictor of the injury of the vehicle passenger caused by the crash event. At the same time, selecting one item of the second data is computationally simple and, therefore, may be executed within a comparatively short period of time, i.e. quickly.

In the system according to the present disclosure the at least one sensor may be configured to measure or detect one or more of an impact force, an angle of collision, a vehicle deformation and a speed at the moment of impact. It is also possible to use a plurality of sensors. Additionally or alternatively, the at least one sensor may form part of an occupant restraint system. Such a sensor may be configured to provide detection results which may be used to determine which airbag should be deployed upon impact. Optionally, also an inflation stage of the airbag may be determined based thereon. Moreover, seat belt activation may be operated based on a detection result of such a sensor. Such a sensor may comprise one or more of a rollover sensor, a front impact sensor, a side impact sensor and a side pressure impact sensor.

According to an example, the output interface is communication interface. Consequently, the predictor of the injury of the vehicle passenger can be communicated to a service entity, e.g. a medical service provider or a traffic regulation authority. Consequently, based on the predictor or prediction, timely and appropriate medical assistance may be provided to the vehicle passenger. Moreover, timely and appropriate actions in traffic management may be taken, e.g. traffic lights may be operated in order to allow the medical service provider to reach the injured vehicle passenger without further delay, intersections may be blocked, etc.

In an example, the system further comprises an input interface for receiving third data indicative of a physical condition of the vehicle passenger. As has been mentioned before, the third data may be provided by a portable electronic device of the vehicle passenger, e.g. a smart phone or smart watch or another wearable device. Additionally or alternatively, the third data may be provided by a passenger monitoring system. Such a system for example comprises a camera or radar unit configured to provide third data. Consequently, the item of the second data is selected not only based on the first data, i.e. the data indicative of the crash event, but also based on the third data. This allows for an even more reliable selection of the most suitable item of the second data, such that a highly precise predictor of the injury of the vehicle passenger may be provided.

According to a fourth aspect, there is provided a vehicle comprising a system of the present disclosure. Using such a vehicle, a good, i.e. sufficiently precise, predictor of the injury of the vehicle passenger caused by the crash event may be provided in case of a crash event. At the same time, this predictor may be provided within a comparatively short period of time, i.e. quickly.

According to a fifth aspect, there is provided a computer program comprising instructions which, when the computer program is executed by a computer, cause the computer to carry out the method of the present disclosure. Thus, using such a computer program, data of historic crash events may be used, wherein in this context, historic means dating from a past time. This data, more precisely the second data, characterizes the crash events as such and additionally comprises at least one associated injury indicator. In simplified words, the second data describe a plurality of crash events and the injuries the respective crash event has caused for a vehicle passenger. The second data may be provided in a database or any other form suitable for selecting one element or item of the second data. As mentioned before, the one item of the second data is selected based on the first data. The first data are indicative of an actual, current and/or imminent crash event. Thus, the first data may be real time data collected by the vehicle undergoing the crash event and/or from the perspective of the vehicle undergoing the crash event. This can also be described in that the selection of the one item of the second data is a function of the first data or at least depends on the first data. Since the at least one injury indicator of the selected historic crash event as described by the selected one item of the second data is used as a predictor of the injury of the vehicle passenger caused by the actual, current and/or imminent crash event, the selection of the one item of the second data is performed such that the selected historic crash event of the second data is similar or at least a suitable approximation of the crash event as described by the first data. Consequently, the injury indicator of the selected historic crash event as described by the selected item of the second data, is a good, i.e. sufficiently precise, predictor of the injury of the vehicle passenger caused by the crash event. At the same time, selecting one item of the second data is computationally simple and, therefore, may be executed within a comparatively short period of time, i.e. quickly.

According to a sixth aspect, there is provided a computer-readable storage medium comprising instructions which, when executed by a data processing device, cause the data processing device to carry out the method of the present disclosure. In this context, the data processing device may be a computer. Thus, using such a computer-readable storage medium, data of historic crash events may be used, wherein in this context, historic means dating from a past time. This data, more precisely the second data, characterizes the crash events as such and additionally comprises at least one associated injury indicator. In simplified words, the second data describe a plurality of crash events and the injuries the respective crash event has caused for a vehicle passenger. The second data may be provided in a database or any other form suitable for selecting one element or item of the second data. As mentioned before, the one item of the second data is selected based on the first data. The first data are indicative of an actual, current and/or imminent crash event. Thus, the first data may be real time data collected by the vehicle undergoing the crash event and/or from the perspective of the vehicle undergoing the crash event. This can also be described in that the selection of the one item of the second data is a function of the first data or at least depends on the first data. Since the at least one injury indicator of the selected historic crash event as described by the selected one item of the second data is used as a predictor of the injury of the vehicle passenger caused by the actual, current and/or imminent crash event, the selection of the one item of the second data is performed such that the selected historic crash event of the second data is similar or at least a suitable approximation of the crash event as described by the first data. Consequently, the injury indicator of the selected historic crash event as described by the selected item of the second data, is a good, i.e. sufficiently precise, predictor of the injury of the vehicle passenger caused by the crash event. At the same time, selecting one item of the second data is computationally simple and, therefore, may be executed within a comparatively short period of time, i.e. quickly.

According to a seventh aspect, there is provided a use of second data indicative of a plurality of historic crash events and at least one associated injury indicator for each of the historic crash events for predicting an injury of a vehicle passenger caused by a crash event. Thus, data of historic crash events may be used, wherein in this context, historic means dating from a past time. This data, more precisely the second data, characterizes the crash events as such and additionally comprises at least one associated injury indicator. In simplified words, the second data describe a plurality of crash events and the injuries the respective crash event has caused for a vehicle passenger. The second data may be provided in a database or any other form suitable for selecting one element or item of the second data. As mentioned before, the one item of the second data is selected based on the first data. The first data are indicative of an actual, current and/or imminent crash event. Thus, the first data may be real time data collected by the vehicle undergoing the crash event and/or from the perspective of the vehicle undergoing the crash event. This can also be described in that the selection of the one item of the second data is a function of the first data or at least depends on the first data. Since the at least one injury indicator of the selected historic crash event as described by the selected one item of the second data is used as a predictor of the injury of the vehicle passenger caused by the actual, current and/or imminent crash event, the selection of the one item of the second data is performed such that the selected historic crash event of the second data is similar or at least a suitable approximation of the crash event as described by the first data. Consequently, the injury indicator of the selected historic crash event as described by the selected item of the second data, is a good, i.e. sufficiently precise, predictor of the injury of the vehicle passenger caused by the crash event. At the same time, selecting one item of the second data is computationally simple and, therefore, may be executed within a comparatively short period of time, i.e. quickly.

It should be noted that the above examples may be combined with each other irrespective of the aspect involved.

These and other aspects of the present disclosure will become apparent from and elucidated with reference to the examples described hereinafter.

### BRIEF DESCRIPTION OF DRAWINGS

Examples of the disclosure will be described in the following with reference to the following drawings.
- Figure 1: shows a vehicle according to a first example of the present disclosure, wherein the vehicle is involved in a crash event, and wherein the vehicle comprises a system according to the present disclosure for predicting an injury of a vehicle passenger and is able to execute a method according to the present disclosure for predicting an injury of a vehicle passenger,
- Figure 2: shows a vehicle which is involved in a crash event, wherein the vehicle is communicatively connected to a cloud computing device configured to execute a method according to the present disclosure for predicting an injury of a vehicle passenger, and
- Figure 3: summarizes the steps of the method according to the present disclosure for predicting an injury of a vehicle passenger.

### DETAILED DESCRIPTION

The Figures are merely schematic representations and serve only to illustrate examples of the disclosure. Identical or equivalent elements are in principle provided with the same reference signs.

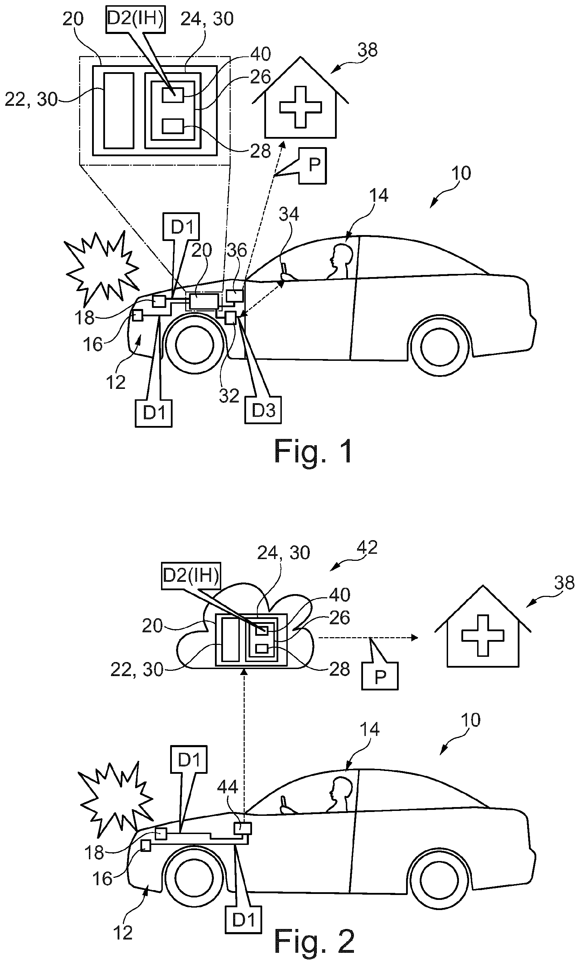

Figure 1 shows a vehicle 10 which is currently involved in a crash event.

The vehicle 10 comprises a system 12 for predicting an injury of a vehicle passenger 14. In the present example, only one vehicle passenger 14 is present in the vehicle 10 for the ease of explanation. This vehicle passenger 14 is the driver of the vehicle 10.

The system 12 comprises a sensor 16 configured for providing first data D1 indicative of a crash event. In the present example, the sensor 16 comprises a strain gauge attached to a structural element of the front portion of the vehicle body. Thus, the sensor 16 is configured to provide a detection result indicative of a strain acting on the vehicle body. Based thereon, an impact force characterizing the crash events may be derived.

It is understood that the sensor 16 may be representative for a plurality of sensors 16. Using such a plurality of sensors, e.g. strain gauges, also an angle of collision and/or a vehicle deformation may be calculated.

The system 12 also comprises a speedometer 18 which is configured to determine a speed of the vehicle 10 and, more precisely, a speed of the vehicle 10 at the moment of impact. This also forms part of the first data D1.

The system 12 also comprises a data processing apparatus 20 to which the sensor 16 and the speedometer 18 are communicatively connected.

The data processing apparatus 20 comprises a data processing unit 22 and a data storage unit 24.

The data storage unit 24 comprises a computer-readable storage medium 26.

On the computer-readable storage medium 26 and, thus, also on the data storage unit 24, there is provided a computer program 28.

The computer program 28 and, thus, also the computer-readable storage medium 26 comprises instructions which, when executed by the data processing unit 22 or more generally speaking a computer, cause the data processing unit 22 or the computer to carry out a method for predicting an injury of the vehicle passenger 14 caused by a crash event.

Consequently, the data processing unit 22 and the data storage unit 24 form means 30 for carrying out the method for predicting an injury of the vehicle passenger 14.

Furthermore, the system 12 comprises an input interface 32 for receiving third data D3 indicative of a physical condition of the vehicle passenger 14.

The input interface 32 is communicatively connected to the data processing apparatus 20. In the present example, the input interface 32 is communicatively coupled to a handheld electronic device 34 of the vehicle passenger 14. The handheld electronic device may be a smart phone.

In the present example, the handheld electronic device 34 comprises an acceleration sensor. Consequently, sensor readings of the acceleration sensor may be provided at the input interface 32.

The system 12 further comprises an output interface 36 communicatively connected to the data processing apparatus 20.

The output interface 36 is configured for providing at least one injury indicator IH as a predictor P of the injury of the vehicle passenger 14. In the present example, the output interface 36 is communicatively coupled to a medical service provider 38 represented schematically only.

The predictor P is provided by executing the method for predicting an injury of the passenger 14. To this end, the data storage unit 24 also comprises a data storage means which in the present example is a database 40 storing second data D2 indicative of a plurality of historic crash events and at least one associated injury indicator IH for each of the historic crash events.

In the present example, the database 40 is stored on the computer-readable storage medium 26.

Thus, in the present example, this database 40 forms part of the data processing apparatus 20. In another example, the database 40 may be provided separate from the data processing apparatus 20. In this case, the database 40 is communicatively connected to the data processing apparatus 20.

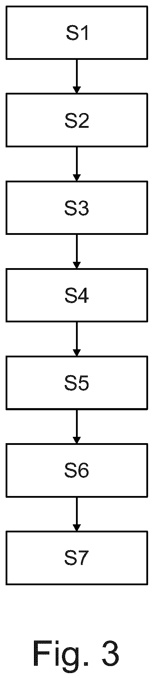

In the following, the method for predicting an injury of the vehicle passenger 14 will be explained in detail. The method steps are summarized in Figure 3.

In a first step S1, first data D1 is obtained. This first data D1 is indicative of the imminent crash event. As has been already explained, in the present example, the first data is provided by the sensor 16 and the speedometer 18.

Thus, in the present example, the first data D1 is indicative of an impact force and a speed of the vehicle 10 at the moment of impact. As has already been explained above, it is also possible that the first data D1 additionally or alternatively is indicative of an angle of collision and/or a vehicle deformation.

Thereafter, in a second step S2, the first data D1 is pre-processed. This means that noise in the first data is reduced. To this end, the first data may be filtered.

Subsequently, in a third step S3, second data D2 is obtained from the database 40.

The second data D2 is indicative of a plurality of historic crash events and indicative of at least one associated injury indicator IH for each of the historic crash events. Similar to the first data D1, also the second data D2 is indicative of an impact force and a speed of the vehicle 10 at the moment of impact. This means that the second data D2 comprises information on impact forces and speeds of the vehicle 10 which have been determined during the historic crash events. As before, it is possible that in other examples, the second data D2 additionally or alternatively is indicative of an angle of collision and/or a vehicle deformation.

The injury indicator IH is indicative of a type of injury, an injury severity and one or more regions of the human body of the vehicle passenger 14 that are affected by the injury.

In the present example, a first type of injuries relates to fractures. A second type of injuries relates to all other injuries.

Moreover, in the present example, the injury severity is described using three classes, wherein a first class relates to light injuries, a second class relates to moderate injuries and a third class relates to severe injuries.

Moreover, the present example, the regions of the human body are legs, torso, arms and head.

The second data is crash test data.

This means that the second data D2 has been generated during crash tests. During these crash tests, vehicles of the same make and model as the vehicle 10 have been tested. Consequently, the database 40 comprises one or more entries for each performed crash test, wherein each entry comprises information on the force of impact, the speed of the vehicle 10, one or more types of injuries, an assessment of the severity of each of the injuries and one or more regions of the human body affected by the injuries.

The database 40 for example comprises 200 entries.

In a subsequent fourth step S4, third data D3 are obtained. As has been explained above, the third data D3 is indicative of a physical condition of the vehicle passenger 14. In the present example, the third data D3 is provided by an acceleration sensor of the handheld electronic device 34 of the vehicle passenger 14. It is assumed that the handheld electronic device 34 is kept in close proximity to the vehicle passenger 14. Consequently, the detection results provided by the acceleration sensor of the handheld electronic device 34 are taken as an indicator of the physical condition of the vehicle passenger 14. In other words, it is assumed that the vehicle passenger 14 and the handheld electronic device 34 are subject to the same acceleration. It is noted that in the context of a crash event, an acceleration may be negative in the sense that it refers to a reduction of speed. Such an acceleration may as well be called a deceleration.

As has been mentioned before, the third data D3 is obtained via the input interface 32.

In a next step, i.e. in a fifth step S5, one item of the second data D2 is selected. This one item is indicative of a selected historic crash event and of an associated injury indicator IH, i.e. a historic injury indicator.

The selection is done based on the first data D1 and based on the third data D3.

In more detail, a first similarity metric M1 is calculated based on the first data D1.

Additionally, a second similarity metric M2 is calculated for each item of the second data D2.

Thus, each second similarity metric M2 is associated with one of the plurality of historic crash events.

Consequently, one item of the second data D2 may be selected. The selected item has a second similarity metric M2 which is closest to the first similarity metric M1. In other words, the item of the second data D2 is selected based on the first similarity metric M1 and the second similarity metric M2.

Since the similarity metrics M1, M2 may comprise a plurality of parameters, a pattern matching technique may be applied for selecting the item of the second data D2. This means that in a case in which the similarity metrics M1, M2 comprise a plurality of parameters, each of the similarity metrics M1, M2 may be expressed or imagined as a pattern. In order to select the item of the second data D2 which describes a historic crash event which has a high or the highest similarity to the imminent crash event, the patterns describing the crash event and the plurality of historic crash events need to be compared and the most similar pattern need to be matched.

In the present example, a very simple pattern may be imagined as comprising a value describing the impact force and a value describing the speed of the vehicle 10.

Also each entry in the database 40 may comprise a value describing the impact force and the value describing the speed of the vehicle 10.

However, there may be no exact match. This means that none of the entries in the database 40 comprises a value describing the impact force equaling the impact force of the crash event and comprises a value describing the speed of the vehicle 10 equaling the speed of the vehicle 10 in the crash event.

In this simple example, the item of the second data D2 may be selected such that a cumulated difference between the value describing the force of impact in the second data D2 and the impact force in the first data D1 and the value describing the speed in the second data D2 and the speed in the first data D1 is minimal.

In the present example, the third data D3 is used to validate the selected item. This means that using the third data D3, a plausibility check is performed on the selected item of the second data.

Afterwards, in a sixth step S6, the at least one injury indicator IH associated with the selected historic crash event is provided as a predictor P of the injury of the vehicle passenger 14 caused by the crash event.

In the present example, this predictor P is provided to the medical service provider 38. Thus, the medical service provider 38 is in a position to quickly provide appropriate medical assistance.

Afterwards, in an optional seventh step S7, the second data D2 may be updated based on the first data D1 and based on at least one associated injury indicator I. The associated injury indicator I may for example be provided by the medical service provider 38. Thus, a new entry is created in the database 40 comprising a value describing the impact force, a value describing the speed of the vehicle 10 and the at least one associated injury indicator.

Thus, in a subsequent use of the database 40, this newly entered entry is a new candidate that may be selected in order to predict an injury of the vehicle passenger 14.

Another example will be explained in connection with Figure 2. In the following, only the differences with respect to the example of Figure 1 will be explained.

In the example of Figure 2, the system 12 for predicting an injury of a vehicle passenger 14 is distributed over the vehicle 10 and a cloud computing device 42.

More precisely, the sensor 16 and the speedometer 18 are still part of the vehicle 10. However, the data processing apparatus 20 forms part of the cloud computing device 42.

Consequently, in the example of Figure 2, the sensor 16 and the speedometer 18 are communicatively connected to the data processing apparatus 20 via a data transmission interface 44. The data is exchanged between the sensor 16, the speedometer 18 and the data processing apparatus 20 in a wireless manner.

Consequently, in a crash event, the method for predicting an injury of the vehicle passenger 14 is executed by the cloud computing device 42.

As before, a predictor P of an injury of the vehicle passenger 14 is provided as a result of the method. Since the method now is executed by the cloud computing device 42, this predictor P is transmitted from the cloud computing device 42 to the medical service provider 38 directly, i.e. not via the vehicle 10.

It is noted that optionally, also the third data D3 may be considered in the example of Figure 2. The explanations provided in connection with Figure 1 apply mutatis mutandis.

It is noted that the example of Figure 1 in which all method steps are executed on the vehicle, i.e. using onboard electronics of the vehicle 10, and the example of Figure 2 in which all method steps are executed on the cloud computing device 42 may be considered as extreme scenarios. It is also possible to distribute the execution of the method steps between the vehicle 10 and the cloud computing device 42, i.e. some steps may be executed by the vehicle 10 and some steps may be executed on the cloud computing device 42.

It is also possible to provide redundancy in the sense that the method may be executed both on the vehicle 10 and on the cloud computing device. The actual location of executing may be determined based on a quality of a data connection between the vehicle 10 and the cloud computing device 42. In an example in which there is no data connection between the vehicle 10 and the cloud computing device 42, all method steps may be executed on the vehicle 10. in an example in which the data connection is of high quality, all method steps may be executed on the cloud computing device 42. In still another example in which the data connection is of medium quality, some steps of the method may be executed on the vehicle 10 and some steps may be executed on the cloud computing device 42.

In summary, all of the above-described examples use second data D2 indicative of a plurality of historic crash events and at least one associated injury indicator IH for each of the historic crash events for predicting an injury of a vehicle passenger 14 caused by a crash event.

As used herein, the phrase "at least one," in reference to a list of one or more entities should be understood to mean at least one entity selected from any one or more of the entities in the list of entities, but not necessarily including at least one of each and every entity specifically listed within the list of entities and not excluding any combinations of entities in the list of entities. This definition also allows that entities may optionally be present other than the entities specifically identified within the list of entities to which the phrase "at least one" refers, whether related or unrelated to those entities specifically identified. Thus, as a non-limiting example, "at least one of A and B" (or, equivalently, "at least one of A or B," or, equivalently "at least one of A and/or B") may refer, in one example, to at least one, optionally including more than one, A, with no B present (and optionally including entities other than B); in another example, to at least one, optionally including more than one, B, with no A present (and optionally including entities other than A); in yet another example, to at least one, optionally including more than one, A, and at least one, optionally including more than one, B (and optionally including other entities). In other words, the phrases "at least one," "one or more," and "and/or" are open-ended expressions that are both conjunctive and disjunctive in operation. For example, each of the expressions "at least one of A, B, and C," "at least one of A, B, or C," "one or more of A, B, and C," "one or more of A, B, or C," and "A, B, and/or C" may mean A alone, B alone, C alone, A and B together, A and C together, B and C together, A, B, and C together, and optionally any of the above in combination with at least one other entity.

Other variations to the disclosed examples can be understood and effected by those skilled in the art in practicing the claimed disclosure, from the study of the drawings, the disclosure, and the appended claims. In the claims the word "comprising" does not exclude other elements or steps and the indefinite article "a" or "an" does not exclude a plurality. A single processor or other unit may fulfill the functions of several items or steps recited in the claims. The mere fact that certain measures are recited in mutually different dependent claims does not indicate that a combination of these measures cannot be used to advantage. A computer program may be stored/distributed on a suitable medium such as an optical storage medium or a solid-state medium supplied together with or as part of other hardware, but may also be distributed in other forms, such as via the Internet or other wired or wireless telecommunication systems. Any reference signs in the claims should not be construed as limiting the scope of the claims.

### LIST OF REFERENCE SIGNS

- 10: vehicle
- 12: system for predicting an injury of a vehicle passenger
- 14: vehicle passenger
- 16: sensor
- 18: speedometer
- 20: data processing apparatus
- 22: data processing unit
- 24: data storage unit
- 26: computer-readable storage medium
- 28: computer program
- 30: means for carrying out the method for predicting an injury of a passenger
- 32: input interface
- 34: handheld electronic device
- 36: output interface
- 38: medical service provider
- 40: database, data storage means
- 42: cloud computing device

- D1: first data
- D2: second data
- D3: second data
- I: injury indicator
- IH: injury indicator of historic crash event
- M1: first similarity metric
- M2: second similarity metric
- P: predictor of an injury
- S1: first step
- S2: second step
- S3: third step
- S4: fourth step
- S5: fifth step
- S6: sixth step
- S7: seventh step

## Claims

1. A method for predicting an injury of a vehicle passenger (14) caused by a crash event, the method comprising
- obtaining first data (D 1) indicative of the crash event (S 1),
- obtaining second data (D2) indicative of a plurality of historic crash events and indicative of at least one associated injury indicator (IH) for each of the historic crash events (S3),
- selecting one item of the second data (D2) indicative of a selected historic crash event based on the first data (D1) (S5), and
- providing the at least one injury indicator (IH) associated with the selected historic crash event as a predictor (P) of the injury of the vehicle passenger (14) caused by the crash event (S6).

2. The method of claim 1, wherein the first data (D1) is indicative of one or more of an impact force, an angle of collision, a vehicle deformation and a speed at the moment of impact and/or
wherein the second data (D2) is indicative of one or more of an impact force, an angle of collision, a vehicle deformation and a speed at the moment of impact for each of the plurality of historic crash events.

3. The method of claim 1 or 2, wherein the injury indicator (IH) is indicative of one or more of a type of injury, an injury severity and one or more regions of the human body.

4. The method of any one of the preceding claims, wherein the second data (D2) is crash test data.

5. The method of any one of the preceding claims, further comprising pre-processing the first data (D1) before selecting one item of the second data (D2) indicative of a selected historic crash event based on the first data (D1) (S2).

6. The method of any one of the preceding claims, wherein selecting one item of the second data (D2) indicative of a selected historic crash event comprises using a pattern matching technique.

7. The method of any one of the preceding claims, further comprising
- obtaining a first similarity metric (M1) based on the first data (D1),
- obtaining a plurality of second similarity metrics (M2) based on the second data (D2), each second similarity metric (M2) being associated with one of the plurality of historic crash events, and
- selecting one item of the second data (D2) indicative of a selected historic crash event based on the first similarity metric (M1) and the second similarity metrics (M2).

8. The method of any one of the preceding claims, further comprising
- obtaining third data (D3) indicative of a physical condition of the vehicle passenger (14) (S4), and
- selecting one item of the second data (D2) indicative of a selected historic crash event based on the third data (D3).

9. The method of any one of the preceding claims, further comprising updating the second data (D2) based on the first data (D1) and based on at least one associated injury indicator (I) (S7).

10. A data processing apparatus (20) comprising means (30) for carrying out the method of any one of the preceding claims.

11. A system (12) for predicting an injury of a vehicle passenger (14) caused by a crash event, the system (12) comprising
- a data processing apparatus (20) of claim 10,
- at least one sensor (16, 18) configured for providing first data (D1) indicative of a crash event, wherein the sensor (16, 18) is communicatively connected to the data processing apparatus (20),
- a data storage means (40) storing second data (D2) indicative of a plurality of historic crash events and at least one associated injury indicator (IH) for each of the historic crash events, wherein the data storage means (40) is communicatively connected to the data processing apparatus (20) or forms part of the data processing apparatus (20), and
- an output interface (36) for providing the at least one injury indicator (IH) as a predictor (P) of the injury of the vehicle passenger (14) caused by the crash event, wherein the output interface (36) is communicatively connected to the data processing apparatus (20).

12. The system of claim 11, further comprising an input interface (32) for receiving third data (D3) indicative of a physical condition of the vehicle passenger (14).

13. A vehicle (10) comprising a system (12) of claim 11 or 12.

14. A computer program (28) comprising instructions which, when the computer program (28) is executed by a computer, cause the computer to carry out the method of claims 1 to 9.

15. A use of second data (D2) indicative of a plurality of historic crash events and at least one associated injury indicator (IH) for each of the historic crash events for predicting an injury of a vehicle passenger (14) caused by a crash event.

## Amended claims

### Amended claims in accordance with Rule 137(2) EPC.

1. A method for predicting an injury of a vehicle passenger (14) caused by a crash event, the method comprising
- obtaining first data (D1) indicative of the crash event (S1),
- obtaining second data (D2) indicative of a plurality of historic crash events and indicative of at least one associated injury indicator (IH) for each of the historic crash events (S3),
- obtaining a first similarity metric (M1) based on the first data (D1),
- obtaining a plurality of second similarity metrics (M2) based on the second data (D2), each second similarity metric (M2) being associated with one of the plurality of historic crash events,
- selecting one item of the second data (D2) indicative of a selected historic crash event based on the first similarity metric (M1) and the second similarity metrics (M2) (S5), and
- providing the at least one injury indicator (IH) associated with the selected historic crash event as a predictor (P) of the injury of the vehicle passenger (14) caused by the crash event (S6),
wherein selecting one item of the second data (D2) indicative of a selected historic crash event comprises using a pattern matching technique, wherein the pattern matching technique comprises a continuous sensor time series similarity pattern matching.

2. The method of claim 1, wherein the first data (D1) is indicative of one or more of an impact force, an angle of collision, a vehicle deformation and a speed at the moment of impact and/or
wherein the second data (D2) is indicative of one or more of an impact force, an angle of collision, a vehicle deformation and a speed at the moment of impact for each of the plurality of historic crash events.

3. The method of claim 1 or 2, wherein the injury indicator (IH) is indicative of one or more of a type of injury, an injury severity and one or more regions of the human body.

4. The method of any one of the preceding claims, wherein the second data (D2) is crash test data.

5. The method of any one of the preceding claims, further comprising pre-processing the first data (D1) before selecting one item of the second data (D2) indicative of a selected historic crash event based on the first data (D1) (S2), wherein the preprocessing comprises manipulating, filtering and/or augmenting.

6. The method of any one of the preceding claims, further comprising
- obtaining third data (D3) indicative of a physical condition of the vehicle passenger (14) (S4), and
- selecting one item of the second data (D2) indicative of a selected historic crash event based on the third data (D3).

7. The method of any one of the preceding claims, further comprising updating the second data (D2) based on the first data (D1) and based on at least one associated injury indicator (I) (S7).

8. A data processing apparatus (20) comprising means (30) for carrying out the method of any one of the preceding claims.

9. A system (12) for predicting an injury of a vehicle passenger (14) caused by a crash event, the system (12) comprising
- a data processing apparatus (20) of claim 8,
- at least one sensor (16, 18) configured for providing first data (D1) indicative of a crash event, wherein the sensor (16, 18) is communicatively connected to the data processing apparatus (20),
- a data storage means (40) storing second data (D2) indicative of a plurality of historic crash events and at least one associated injury indicator (IH) for each of the historic crash events, wherein the data storage means (40) is communicatively connected to the data processing apparatus (20) or forms part of the data processing apparatus (20), and
- an output interface (36) for providing the at least one injury indicator (IH) as a predictor (P) of the injury of the vehicle passenger (14) caused by the crash event, wherein the output interface (36) is communicatively connected to the data processing apparatus (20).

10. The system of claim 9, further comprising an input interface (32) for receiving third data (D3) indicative of a physical condition of the vehicle passenger (14).

11. A vehicle (10) comprising a system (12) of claim 9 or 10.

12. A computer program (28) comprising instructions which, when the computer program (28) is executed by a computer, cause the computer to carry out the method of claims 1 to 7.
